# EUROPEAN PATENT SPECIFICATION

(11) **EP 4 245 215 B1**
(45) Date of publication and mention of the grant of the patent: **16.04.2025**
(21) Application number: 21891005.7
(22) Date of filing: 02.11.2021
(51) Int. Cl.: A61B 5/055, G01R 33/34, G01R 33/385, G01R 33/3815

(54) **SUPERCONDUCTING MAGNETIC RESONANCE IMAGING SYSTEM FOR LIMB**
SUPRALEITENDES MAGNETRESONANZBILDGEBUNGSSYSTEM FÜR GLIEDMASSEN
SYSTÈME D'IMAGERIE PAR RÉSONANCE MAGNÉTIQUE SUPRACONDUCTRICE POUR MEMBRE

(30) Priority: 13.11.2020 CN 202011264531
(43) Date of publication of application: 20.09.2023
(73) Proprietor: Shandong Aoxin Medical Technology Co., Ltd., Weifang, Shandong 261061 (CN)
(72) Inventor: LI, Peiyong, Weifang, Shandong 261061 (CN); LIU, Yu, Weifang, Shandong 261061 (CN); CHENG, Dongqin, Weifang, Shandong 261061 (CN); LU, Zhiqiang, Weifang, Shandong 261061 (CN); LU, Yao, Weifang, Shandong 261061 (CN)
(74) Representative: Prinz & Partner mbB
(86) International application number: PCT/CN2021/128049
(87) International publication number: WO 2022/100478

(56) References cited:
- WO-A1-2019/103402
- WO-A1-2019/103402
- CN-A- 1 736 329
- CN-A- 103 549 957
- CN-A- 105 044 633
- CN-A- 107 305 242
- CN-A- 108 107 387
- CN-A- 108 447 644
- CN-A- 108 447 644
- CN-A- 111 643 081
- CN-A- 111 643 081
- CN-A- 112 075 935
- CN-U- 210 494 071
- CN-U- 211 375 028
- US-A- 5 664 568
- US-A1- 2017 143 203
- US-A1- 2020 025 846

## Description

This application claims the benefit of priority to Chinese Patent Application No. 202011264531.3, titled "SUPERCONDUCTING MAGNETIC RESONANCE IMAGING SYSTEM FOR EXTREMITY", filed with the China National Intellectual Property Administration on November 13, 2020.

### FIELD

The present application relates to the technical field of magnetic resonance imaging, and in particular to a superconducting magnetic resonance imaging system for extremity.

### BACKGROUND

Magnetic resonance apparatus are currently core apparatus in the field of global medical imaging diagnosis. Most of the commonly used magnetic resonance apparatus on the market are whole-body 1.5T and 3.0T magnetic resonance apparatus. The whole-body magnetic resonance apparatus generally has disadvantages of large volume, heavy weight, large occupied area, high cost, and high maintenance cost. For example, the 3.0T apparatus weighs about 7000Kg and occupies an area of about 35 square meters. In terms of imaging, limited by hardware performance, the imaging of small joints is not clear and the imaging speed is slow. In terms of the comfort level, it requires the whole body of the subject to enter the examination hole, which may cause claustrophobia, and the apparatus is noisy.

Therefore, a technical problem to be solved by those skilled in the art is to provide a magnetic resonance apparatus which occupies a small space and provides a high comfort level.
US 5664568 A discloses a magnetic resonance imaging system with an insertable head and neck coil, where multiple primary magnetic coils generate a magnetic field along a longitudinal or z-axis of a central bore. A whole body gradient coil assembly includes coils mounted along the bore for generating gradient magnetic fields. A whole body radio frequency coil is mounted inside the gradient coil assembly. A whole body radio frequency shield is mounted between the whole body RF coil and the gradient coil assembly. An insertable radio frequency coil is removably mounted in the bore in an examination region defined around an isocenter of the magnet. A sequence controller controls gradient amplifiers connected with the gradient coil assembly for causing the generation of the gradient magnetic fields at appropriate times during the selected gradient sequence and a digital transmitter which causes a selected one of the whole body and insertable radio frequency coils to generate radio frequency field pulses at times appropriate to the selected sequence.
US 20170143203 A1 discloses an MRI coil base apparatus that is configured for use with interchangeable coils that are attachable and detachable to and from the MRI coil base apparatus. An interchangeable coil may be attached to the base for an MRI procedure and then may be removed and replaced by a different interchangeable coil for a subsequent MRI procedure. An example coil may be used with an example base that is configured for use with interchangeable attachable and detachable coils. Connector may mechanically or electrically connect coil to the MRI coil base apparatus. The MRI coil is configured to be mechanically and electrically coupled to the base. The coil may be a member of a set of different MRI coils that can be attached to and detached from base

CN211375028 U discloses an extremity MRI system with a body coil and a surface coil and a connection device which enables to mount the surface coil within the body coil.

### SUMMARY

In view of this, an object of the present application is to provide a superconducting magnetic resonance imaging system for an extremity which occupies a small space and provides a high comfort level during examination.

In order to achieve the above object, the following technical solutions are provided according to the present application.

A superconducting magnetic resonance imaging system for an extremity includes a magnetic system, a radio frequency system and a console, the magnetic system includes a loop-shaped main housing, the radio frequency system includes loop-shaped radio frequency coils, the radio frequency coils include an extremity coil and at least two types of extremity-part coils, the extremity coil is a transmit-receive coil, and each of the extremity-part coils is a receive-only coil configured for only receiving signals, the extremity coil is arranged on an inner loop-shaped surface of the main housing, each of the extremity-part coils is configured to be switchably connected to an inner loop-shaped surface of the extremity coil and the radio frequency coils are communicatively connected with the console in a detachable manner; where
the radio frequency coils further include a knee coil for imaging of a knee, the knee coil is a transmit-receive coil, the extremity coil and the knee coil are configured to be switchably connected to the inner loop-shaped surface of the main housing, and an inner diameter of the knee coil is smaller than an inner diameter of the extremity coil; and
the extremity-part coils include a hand coil for imaging of a hand, the hand coil includes a loop-shaped hand coil main body and a hand coil support board which is arranged at one axial side of the hand coil main body, an inner loop-shaped surface of the hand coil main body is formed by a C-shaped surface and a main body flat surface connected to an opening of the C-shaped surface, and the hand coil support board is a flat surface and is arranged coplanar with the main body flat surface.

Preferably, a diameter of the inner loop-shaped surface of the extremity coil ranges from 175 mm to 185 mm, and a diameter of an inner diameter surface of the knee coil ranges from 155 mm to 165 mm.

Preferably, the extremity-part coils include at least one of an elbow coil for imaging of an elbow and an ankle coil for imaging of an ankle.

Preferably, the elbow coil includes a loop-shaped elbow coil main body and two elbow coil support boards arranged at two axial sides of the elbow coil main body.

Preferably, the ankle coil includes a loop-shaped ankle coil main body and a loop-shaped ankle coil support board, one port of the ankle coil main body is butted to one port of the ankle coil support board, and an included angle is formed between a centerline of the ankle coil main body and a centerline of the ankle coil support board.

Preferably, the superconducting magnetic resonance imaging system for the extremity further includes a scanning chair which is made of a non-magnetic material and is arranged outside the main housing, the scanning chair includes a seat plate, a footrest and a backrest which are arranged at two sides of the seat plate, and an angle of the foot rest and an angle of the backrest are adjustable relative to the seat plate.

Preferably, the magnetic system further includes a superconducting magnet arranged in the main housing, a magnetic field intensity of the superconducting magnet is 3.0T, and a 3.0T extremity-dedicated pulse sequence is built in a controller of the console.

Preferably, the magnetic system further includes a gradient system, the gradient system includes a gradient coil, the gradient coil has an annular structure with a length less than or equal to 640 mm, an outer diameter less than or equal to 294 mm and an inner diameter greater than or equal to 210 mm.

During examination, a suitable extremity-part coil is selected and is mounted in the extremity coil, so as to perform examination to the corresponding extremity part. The extremity coil transmits a signal to the extremity part to be examined but does not receive an external signal, a return signal is received by the extremity-part coil, and then data analysis and imaging are performed by the console. In addition, due to the difference in body shape of the examination subject, for an overweight examination subject, the extremity-part coil may not be mounted in the extremity coil, instead, the extremity coil may be directly used for examination. For example, a knee of the examination object extends into the extremity coil, the extremity coil transmits and receives signals, and then the console performs data analysis and imaging.

It can be seen that during imaging, only the extremity part to be examined is required to be placed into the corresponding radio frequency coil, while other parts of the body are still outside the magnet, which avoids the disadvantages of whole-body magnetic resonance such as causing claustrophobia, difficulty in placing the upper extremity part to be examined in the center of the imaging area and high noise, and has a high comfort level. In addition, since the apparatus does not need to accommodate the whole human body, the occupied space is small, and the manufacturing cost of the apparatus is reduced due to the decrease of the size.

### BRIEF DESCRIPTION OF THE DRAWINGS

For more clearly illustrating embodiments of the present application or the technical solutions in the conventional technology, drawings to be used in the description of the embodiments or the conventional technology will be briefly described hereinafter. Apparently, the drawings in the following description are only embodiments of the present application, for those skilled in the art, other drawings may be obtained based on the provided drawings without any creative work.
FIG. 1 is a view showing the structure of an extremity coil in an imaging system according to the present application from a first perspective;
FIG. 2 is a view showing the structure of the extremity coil in the imaging system according to the present application from a second perspective;
FIG. 3 is a view showing the structure of a knee coil in the imaging system according to the present application from a first perspective;
FIG. 4 is a view showing the structure of the knee coil in the imaging system according to the present application from a second perspective;
FIG. 5 is a view showing the structure of an ankle coil in the imaging system according to the present application from a first perspective;
FIG. 6 is a view showing the structure of the ankle coil in the imaging system according to the present application from a second perspective;
FIG. 7 is a view showing the structure of a hand coil in the imaging system according to the present application from a first perspective;
FIG. 8 is a view showing the structure of the hand coil in the imaging system according to the present application from a second perspective;
FIG. 9 is a view showing the structure of an elbow coil in the imaging system according to the present application;
FIG. 10 is a view showing the structure of a scanning chair in the imaging system according to the present application;
FIG. 11 is a view showing the structure of a console in the imaging system according to the present application;
FIG. 12 is a view showing the structure of a gradient system in the imaging system according to the present application;
FIG. 13 is a view showing the structure of a magnetic system in the imaging system according to the present application;
FIG. 14 is a structural view showing that the extremity coil is mounted in the magnetic system in the imaging system according to the present application;
FIG. 15 is a structural view showing that the extremity coil and the ankle coil are mounted in the magnetic system in the imaging system according to the present application; and
FIG. 16 is a structural view showing that the ankle coil is mounted in the extremity coil in the imaging system according to the present application.

Reference numerals in the drawings:

| | | | |
|---|---|---|---|
| 1 | magnetic system, | 2 | main housing, |
| 3 | gradient system, | 4 | extremity coil, |
| 5 | knee coil, | 6 | hand coil, |
| 61 | hand coil main body, | 62 | hand coil support board, |
| 7 | elbow coil, | 71 | elbow coil main body, |
| 72 | elbow coil support board, | 8 | ankle coil, |
| 81 | ankle coil main body, | 82 | ankle coil support board, |
| 9 | scanning chair, | 91 | footrest, |
| 92 | backrest, | 93 | seat plate, |
| 10 | console. | | |

### DETAILED DESCRIPTION OF THE EMBODIMENTS

Technical solutions in the embodiments of the present application are clearly and completely described hereinafter in conjunction with the drawings in the embodiments of the present application. Apparently, the embodiments described in the following are only some embodiments of the present application, rather than all embodiments. Based on the embodiments in the present application, all of the other embodiments encompassed by the claims fall within the protection scope of the present invention.

A core according to the present application is to provide a superconducting magnetic resonance imaging system for an extremity, which occupies a small space and provides a high comfort level during examination.

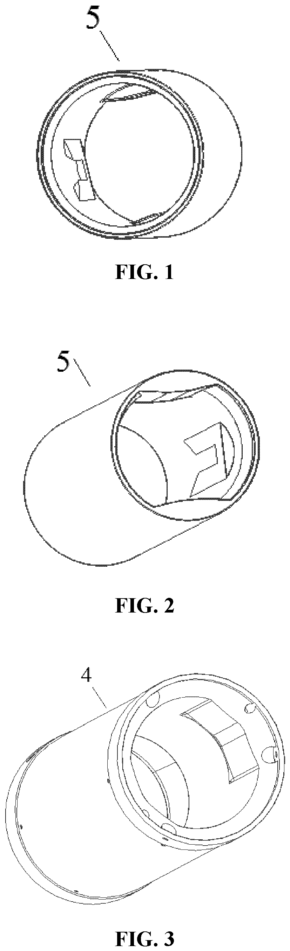

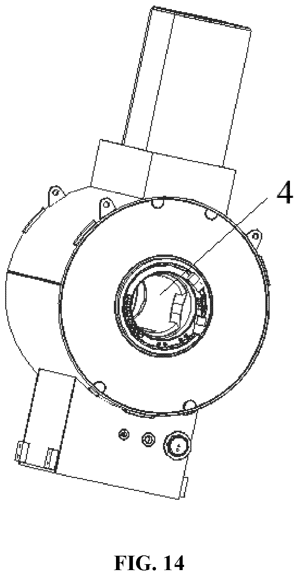

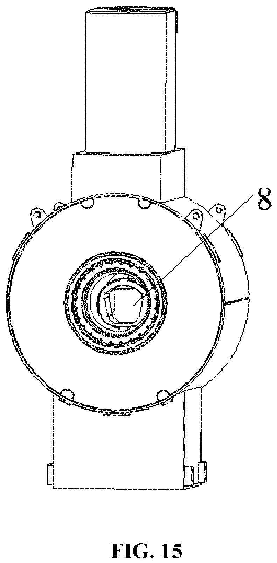

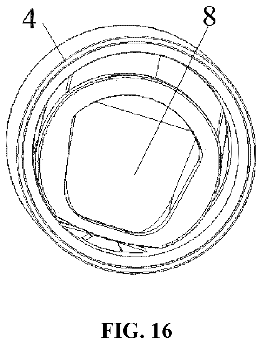

The superconducting magnetic resonance imaging system for the extremity according to a specific embodiment of the present application, referring to FIGS. 1 to 16, includes a magnetic system 1, a radio frequency system and a console 10.

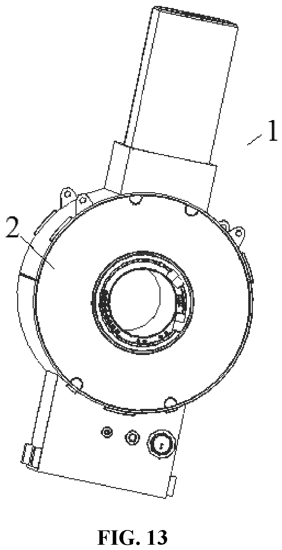

The magnetic system 1 includes a loop-shaped main housing 2, referring to FIG. 13, an inner loop-shaped surface of the main housing 2 forms an examination hole, and a diameter of the hole is greater than or equal to 300 mm. In addition, an outer loop-shaped surface of the main housing 2 has an outer diameter less than or equal to 672 mm and a length less than or equal to 620 mm, which has a compact structure, and can meet requirements of sizes of hands, feet, elbows, knees and positioning of extremities during examination. A superconducting magnet is arranged in the main housing 2, which specifically is a superconducting coil structure with a magnetic field intensity reaching 3.0T, an imaging area greater than 160 mm, a magnetic field uniformity less than 0.6 ppm, a magnetic field stability less than 0.05 ppm, liquid helium consumption less than or equal to 80 L, and no volatilization of liquid helium, which has high imaging resolution, high signal-to-noise ratio and good image quality.

The radio frequency system includes at least two types of loop-shaped radio frequency coils, which specifically includes an extremity coil 4 and at least two types of extremity-part coils. The extremity coil 4 is a transmit-receive coil, which can receive and transmit signals. The extremity-part coils are receiver coils which only receive signals. The extremity coil 4 is arranged on an inner loop-shaped surface of the main housing 2.

Each extremity-part coil is configured to be switchably connected to an inner loop-shaped surface of the extremity coil 4, that is, each extremity-part coil is detachably connected to the extremity coil 4, and when switching is required, the currently used extremity-part coil is detached and replaced by another type of extremity-part coil. The extremity-part coils are configured for examination of corresponding different extremity parts, for example, for examination of corresponding hands, elbows, ankles and other parts respectively.

During examination, a suitable extremity-part coil is selected and is mounted in the extremity coil 4, so as to perform examination to the corresponding extremity part. The extremity coil 4 transmits a signal to the extremity part to be examined but does not receive an external signal, a return signal is received by the extremity-part coil, and then data analysis and imaging are performed by the console 10. In addition, due to the difference in body shape of the examination subject, for an overweight examination subject, the extremity-part coil may not be mounted in the extremity coil 4, instead, the extremity coil 4 may be directly used for examination. For example, a knee of the examination object extends into the extremity coil 4, the extremity coil 4 transmits and receives signals, and then the console 10 performs data analysis and imaging.

It can be seen that in this embodiment, during imaging, only the extremity-part to be examined is required to be placed into the corresponding radio frequency coil, while other parts of the body are still outside the magnet, which avoids the disadvantages of whole-body magnetic resonance such as causing claustrophobia, difficulty in placing the upper extremity-part to be examined in the center of the imaging area and high noise, and has a high comfort level. In addition, since the apparatus does not need to accommodate the whole human body, the occupied space is small, and the manufacturing cost of the apparatus is reduced due to the decrease of the size.

Furthermore, the radio frequency coils further include a knee coil 5, and the knee coil 5 is a transmit-receive coil. The extremity coil 4 and the knee coil 5 are switchably connected to the inner loop-shaped surface of the main housing 2, that is, the extremity coil 4 and the knee coil 5 are detachably connected to the main housing 2, and when one of the extremity coil 4 and the knee coil 5 is detached, the other one can be connected.

An inner loop-shaped surface of the knee coil 5 and an inner loop-shaped surface of the extremity coil 4 are both cylindrical surfaces, and an inner diameter of the knee coil 5 is smaller than an inner diameter of the extremity coil 4. Optionally, a diameter of the inner loop-shaped surface of the extremity coil 4 ranges from 175 mm to 185 mm, which is specifically 180 mm, and a diameter of an inner diameter surface of the knee coil 5 ranges from 155 mm to 165 mm, which is specifically 160 mm. In addition, other positioning structures may also be provided on the inner loop-shaped surfaces of the knee coil 5 and the extremity coil 4, so as to position extremity parts or be connected to other coils.

In this embodiment, during the knee examination, if the examination subject is overweight, the extremity coil 4 may be used for knee examination; if the examination subject is thin and the leg can extend into the knee coil 5, the extremity coil 4 may be removed from the main housing 2, and the knee coil 5 may be mounted in the main housing 2 for examination. Thus, knee imaging may be adaptively realized in consideration of the body shape of the examination subject, which has high applicability.

Further, the extremity-part coils include a hand coil 6 and may further include an elbow coil 7 and an ankle coil 8. Preferably, the hand coil 6, the elbow coil 7 and the ankle coil 8 are provided in a manner of simulating shapes of the hands, elbows and ankles respectively, which can further improve the adaptability to the extremity part to be examined. During examination, the extremity-part coil can be closer to the extremity surface, a better signal-to-noise ratio is obtained, and thus the imaging is clearer.

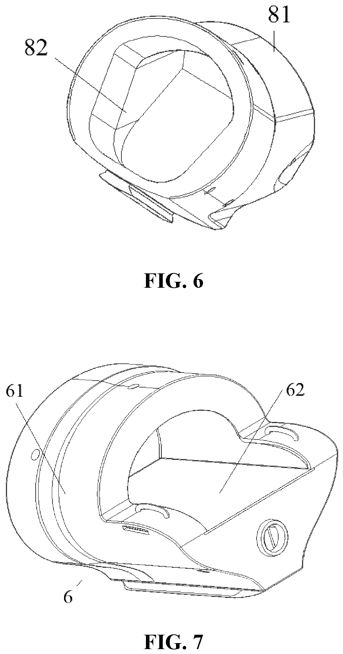

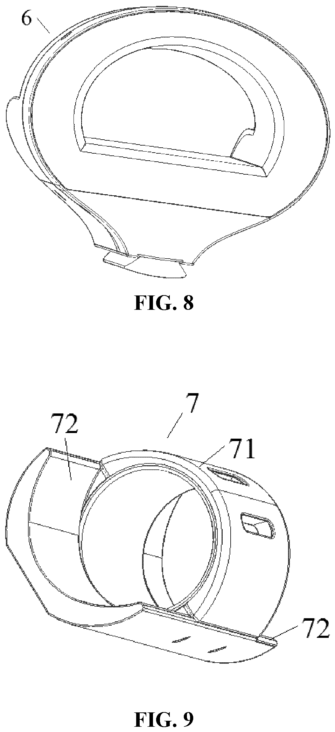

Further, referring to FIG. 7 and FIG. 8, the hand coil 6 includes a loop-shaped hand coil main body 61 and a hand coil support board 62 arranged at one axial side of the hand coil main body 61, an inner loop-shaped surface of the hand coil main body 61 is formed by a C-shaped surface and a main body flat surface connected to an opening of the C-shaped surface, and the hand coil support board 62 is a flat surface and is arranged coplanar with the main body flat surface. During use, the hand coil support board 62 can support the arm, and the hand extends into the hand coil main body 61 for examination, which has a good comfort level during the examination, and can keep the arm stable.

Further, referring to FIG. 9, the elbow coil 7 includes a loop-shaped elbow coil main body 71 and two elbow coil support boards 72 arranged at two axial sides of the elbow coil main body 71. The two elbow coil support boards 72 can support the parts of the arm located at two sides of the elbow respectively, which can improve the stability of arm placement, and thereby avoiding arm soreness during the examination.

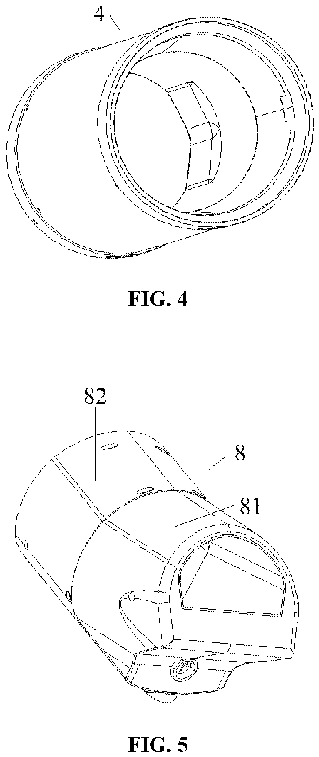

Further, referring to FIG. 5 and FIG. 6, the ankle coil 8 includes a loop-shaped ankle coil main body 81 and a loop-shaped ankle coil support board 82, one port of the ankle coil main body 81 is butted to one port of the ankle coil support board 82, and an included angle is formed between a centerline of the ankle coil main body 81 and a centerline of the ankle coil support board 82. During ankle examination, the centerline of the ankle coil support board 82 is substantially horizontal, the ankle coil main body 81 is gradually inclined upward from the joint with the ankle coil support board 82 toward the other end of the ankle coil main body 81, the foot extends from the ankle coil support board 82 into the ankle coil main body 81, the leg may rest on the ankle coil support board 82, the foot may rest on a bottom of the inner loop-shaped surface of the ankle coil main body 81 which is inclined, and the ankle may be located at a junction between the ankle coil support board 82 and the ankle coil main body 81, so that the leg has a high comfort level during the examination, and the ankle is positioned well during examination.

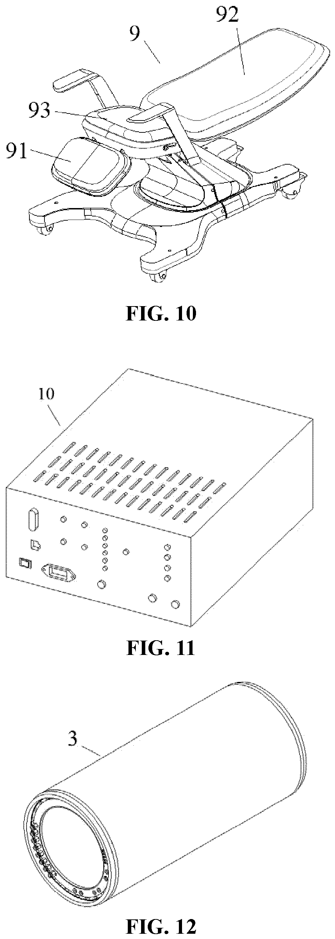

Further, referring to FIG. 10, the imaging system further includes a scanning chair 9 which is made of a non-magnetic material and is arranged outside the main housing 2, the scanning chair 9 includes a seat plate 93, a footrest 91 and a backrest 92 which are arranged at two sides of the seat plate 93, and an angle of the footrest 91 and an angle of the backrest 92 are adjustable relative to the seat plate 93. Specifically, linkage adjustment of the backrest 92 and the seat plate 93 can be performed through an adjustment structure. By adjusting the angles of the backrest 92 and the footrest 91, the scanning chair 9 can be in a seat state or a bed state, so that the examination subject can sit or lie on the scanning chair 9 for examination. When the scanning chair 9 is adjusted to the seat state, the examination subject can sit on the scanning chair 9 in a sitting posture for lower extremity scanning and imaging and when the scanning chair 9 is adjusted to the bed state, the examination subject can lie on the scanning chair 9 in a lying posture for upper extremity scanning and imaging. In addition, the scanning chair 9 further includes a bracket which is fixed under the seat plate 93 and a roller arranged under the bracket, so as to realize functions such as rotation and movement of the scanning chair 9, which facilitates adjustment of the position of the scanning chair 9.

Further, referring to FIG. 10, a software of a controller of the console 10 employs a 3.0T extremity-dedicated imaging sequence, which, compared with the conventional imaging sequence, is optimized and adjusted in terms of gradient waveform, climbing time and pulse width, which further gives play to the hardware performance of the product, so that the imaging quality is better and the scanning speed is faster. In addition, the console 10 is specifically a dedicated magnetic resonance console with four channels for reception and two channels for transmission. In order to realize the specific imaging of joints and bone cortex, the time resolution reaches 4 ns and the phase accuracy reaches 0.001 degrees. The console 10 has a split structure, which is more convenient for future upgrades and saves the consumption of radio frequency signal lines.

Further, referring to FIG. 12, the magnetic system 1 further includes a gradient system 3, the gradient system 3 includes a gradient coil, which may be specifically arranged between the inner loop-shaped surface of the main housing 2 and the radio frequency coil. The gradient coil has an annular structure with a length less than or equal to 640 mm, an outer diameter less than or equal to 294 mm, and an inner diameter greater than or equal to 210 mm. The gradient coil is a small-diameter extremity gradient coil, which can just meet the size requirements of human extremities, so that the knee or elbow of the human can reach the center of the imaging area. In addition, the gradient coil is a high-performance gradient coil with high gradient field intensity, high switching rate and low eddy current value, where the gradient intensity is 110 mT/m, the switching rate is 500T/m/s, the nonlinearity is less than 2%, the eddy current value is less than 1%, and the gradient intensity and switching rate are respectively 2 to 3 times of the whole-body 3.0T apparatus, thus the gradient coil has better performance in efficiency, gradient field intensity and switching rate, which also has small operating current and low noise, and thereby the requirements of high-resolution imaging and fast imaging are well satisfied.

According to the imaging system of this embodiment, core components of each subsystem are provided according to the specialized requirements of extremity imaging, and the performance is greatly improved, which can not only meet the disease diagnosis requirements of high-resolution and fast imaging, but also meet the scientific research needs of the advanced imaging functions.

It should be noted that when an element is referred to as being "fixed" to another element, it may be directly fixed to the another element or an intermediate element may be provided. All technical and scientific terms used in the present application have the same meaning as commonly understood by one of ordinary skill in the art to which this disclosure belongs, unless otherwise defined. The terms in the present application are used to describe specific embodiments, which are not intended to limit the present application.

The above embodiments are described in a progressive manner, each of the embodiments is mainly focused on describing its differences from other embodiments, and reference may be made among these embodiments with respect to the same or similar parts.

The superconducting magnetic resonance imaging system for the extremity according to the present application has been described in detail hereinbefore. The principle and embodiments of the present application are described through specific examples herein. The description of the above-described embodiments is merely used to facilitate understanding the method and core idea of the present application. It should be noted that, for those skilled in the art, many improvements and modifications may be further made to the present application without departing from the principle of the present application, and these improvements and modifications also fall within the protection scope of claims of the present application. In any case, the scope of the invention is defined by the claims.

## Claims

1. A superconducting magnetic resonance imaging system for an extremity, comprising a magnetic system (1), a radio frequency system and a console (10), wherein the magnetic system (1) comprises a loop-shaped main housing (2), the radio frequency system comprises loop-shaped radio frequency coils, wherein
the radio frequency coils comprise an extremity coil (4) and at least two types of extremity-part coils, the extremity coil (4) is a transmit-receive coil, and each of the extremity-part coils is a receive-only coil configured for only receiving signals; the extremity coil (4) is arranged on an inner loop-shaped surface of the main housing (2), each of the extremity-part coils is configured to be switchably connected to an inner loop-shaped surface of the extremity coil (4), and the radio frequency coils are communicatively connected with the console (10) in a detachable manner; wherein
the radio frequency coils further comprise a knee coil (5) for imaging of a knee, the knee coil (5) is a transmit-receive coil, the extremity coil (4) and the knee coil (5) are configured to be switchably connected to the inner loop-shaped surface of the main housing (2), and an inner diameter of the knee coil (5) is smaller than an inner diameter of the extremity coil (4); and
the extremity-part coils comprise a hand coil (6) for imaging of a hand, the hand coil (6) comprises a loop-shaped hand coil main body (61) and a hand coil support board (62) which is arranged at one axial side of the hand coil main body (61), an inner loop-shaped surface of the hand coil main body (61) is formed by a C-shaped surface and a main body flat surface connected to an opening of the C-shaped surface, and the hand coil support board (62) is a flat surface and is arranged coplanar with the main body flat surface.

2. The superconducting magnetic resonance imaging system for an extremity according to claim 1, wherein a diameter of the inner loop-shaped surface of the extremity coil (4) ranges from 175 mm to 185 mm, and a diameter of an inner diameter surface of the knee coil (5) ranges from 155 mm to 165 mm.

3. The superconducting magnetic resonance imaging system for an extremity according to claim 1, wherein the extremity-part coils further comprise at least one of an elbow coil (7) for imaging of an elbow and an ankle coil (8) for imaging of an ankle.

4. The superconducting magnetic resonance imaging system for an extremity according to claim 3, wherein the elbow coil (7) comprises a loop-shaped elbow coil main body (71) and two elbow coil support boards arranged at two axial sides of the elbow coil main body (71).

5. The superconducting magnetic resonance imaging system for an extremity according to claim 3, wherein the ankle coil (8) comprises a loop-shaped ankle coil main body (81) and a loop-shaped ankle coil support board (82), one port of the ankle coil main body (81) is butted to one port of the ankle coil support board (82), and an included angle is formed between a centerline of the ankle coil main body (81) and a centerline of the ankle coil support board (82).

6. The superconducting magnetic resonance imaging system for an extremity according to any one of claims 1 to 5, further comprising a scanning chair (9) which is made of a non-magnetic material and is arranged outside the main housing (2), wherein the scanning chair (9) comprises a seat plate (93), a footrest (91) and a backrest (92) which are arranged at two sides of the seat plate (93), and an angle of the footrest (91) and an angle of the backrest (92) are adjustable relative to the seat plate (93).

7. The superconducting magnetic resonance imaging system for an extremity according to claim 6, wherein the magnetic system (1) further comprises a superconducting magnet arranged in the main housing (2), a magnetic field intensity of the superconducting magnet is 3.0T, and a 3.0T extremity-dedicated imaging sequence is built in a controller of the console (10).

8. The superconducting magnetic resonance imaging system for an extremity according to claim 6, wherein the magnetic system (1) further comprises a gradient system (3), the gradient system (3) comprises a gradient coil, the gradient coil has an annular structure with a length less than or equal to 640 mm, an outer diameter less than or equal to 294 mm and an inner diameter greater than or equal to 210 mm.

## Patentansprüche

1. Supraleitendes Magnetresonanzbildgebungssystem für eine Extremität, mit einem Magnetsystem (1), einem Hochfrequenzsystem und einem Bedienpult (10), wobei das Magnetsystem (1) ein schleifenförmiges Hauptgehäuse (2) umfasst, wobei das Hochfrequenzsystem schleifenförmige Hochfrequenzspulen umfasst, wobei
die Hochfrequenzspulen eine Extremitätenspule (4) und mindestens zwei Arten von Extremitätenteilspulen umfassen, die Extremitätenspule (4) eine Sende-Empfangs-Spule ist und jede der Extremitätenteilspulen eine Nur-Empfangs-Spule ist, die nur zum Empfangen von Signalen eingerichtet ist, die Extremitätenspule (4) auf einer schleifenförmigen Innenfläche des Hauptgehäuses (2) angeordnet ist, jede der Extremitätenteilspulen so eingerichtet ist, dass sie mit einer schleifenförmigen Innenfläche der Extremitätenspule (4) schaltbar verbunden ist, und die Hochfrequenzspulen lösbar mit dem Bedienpult (10) kommunikationsmäßig verbunden sind, wobei
die Hochfrequenzspulen ferner eine Kniespule (5) zur Bildgebung eines Knies umfassen, die Kniespule (5) eine Sende-Empfangs-Spule ist, die Extremitätenspule (4) und die Kniespule (5) so eingerichtet sind, dass sie schaltbar mit der schleifenförmigen Innenfläche des Hauptgehäuses (2) verbunden sind, und ein Innendurchmesser der Kniespule (5) kleiner als ein Innendurchmesser der Extremitätenspule (4) ist und
die Extremitätenteilspulen eine Handspule (6) zur Bildgebung einer Hand umfassen, die Handspule (6) einen schleifenförmigen Handspulenhauptkörper (61) und eine Handspulentragplatte (62) umfasst, die an einer axialen Seite des Handspulenhauptkörpers (61) angeordnet ist, eine schleifenförmige Innenfläche des Handspulenhauptkörpers (61) von einer C-förmigen Fläche und einer ebenen Hauptkörperfläche gebildet ist, die mit einer Öffnung der C-förmigen Fläche verbunden ist, und die Handspulentragplatte (62) eine ebene Fläche ist und koplanar zu der ebenen Hauptkörperfläche angeordnet ist.

2. Supraleitendes Magnetresonanzbildgebungssystem für eine Extremität nach Anspruch 1, wobei ein Durchmesser der schleifenförmigen Innenfläche der Extremitätenspule (4) im Bereich von 175 mm bis 185 mm liegt und ein Durchmesser einer Innendurchmesserfläche der Kniespule (5) im Bereich von 155 mm bis 165 mm liegt.

3. Supraleitendes Magnetresonanzbildgebungssystem für eine Extremität nach Anspruch 1, wobei die Extremitätenteilspulen ferner eine Ellenbogenspule (7) zur Bildgebung eines Ellenbogens und/oder eine Knöchelspule (8) zur Bildgebung eines Knöchels umfassen.

4. Supraleitendes Magnetresonanzbildgebungssystem für eine Extremität nach Anspruch 3, wobei die Ellenbogenspule (7) einen schleifenförmigen Ellenbogenspulenhauptkörper (71) und zwei Ellenbogenspulentragplatten umfasst, die an zwei axialen Seiten des Ellenbogenspulenhauptkörpers (71) angeordnet sind.

5. Supraleitendes Magnetresonanzbildgebungssystem für eine Extremität nach Anspruch 3, wobei die Knöchelspule (8) einen schleifenförmigen Knöchelspulenhauptkörper (81) und eine schleifenförmige Knöchelspulentragplatte (82) umfasst, eine Öffnung des Knöchelspulenhauptkörpers (81) an eine Öffnung der Knöchelspulentragplatte (82) anstößt und zwischen einer Mittellinie des Knöchelspulenhauptkörpers (81) und einer Mittellinie der Knöchelspulentragplatte (82) ein eingeschlossener Winkel gebildet ist.

6. Supraleitendes Magnetresonanzbildgebungssystem für eine Extremität nach einem der Ansprüche 1 bis 5, das ferner einen Abtaststuhl (9) umfasst, der aus einem nichtmagnetischen Material besteht und außerhalb des Hauptgehäuses (2) angeordnet ist, wobei der Abtaststuhl (9) eine Sitzplatte (93), eine Fußstütze (91) und eine Rückenlehne (92) umfasst, die an zwei Seiten der Sitzplatte (93) angeordnet sind, und ein Winkel der Fußstütze (91) und ein Winkel der Rückenlehne (92) relativ zur Sitzplatte (93) verstellbar sind.

7. Supraleitendes Magnetresonanzbildgebungssystem für eine Extremität nach Anspruch 6, wobei das Magnetsystem (1) ferner einen supraleitenden Magneten umfasst, der in dem Hauptgehäuse (2) angeordnet ist, eine Magnetfeldstärke des supraleitenden Magneten 3,0 T beträgt und eine extremitätendedizierte Bildgebungssequenz von 3,0 T in eine Steuerung des Bedienpults (10) eingebaut ist.

8. Supraleitendes Magnetresonanzbildgebungssystem für eine Extremität nach Anspruch 6, wobei das Magnetsystem (1) ferner ein Gradientensystem (3) umfasst, das Gradientensystem (3) eine Gradientenspule umfasst, die Gradientenspule eine Ringstruktur mit einer Länge kleiner oder gleich 640 mm, einem Außendurchmesser kleiner oder gleich 294 mm und einem Innendurchmesser größer oder gleich 210 mm aufweist.

## Revendications

1. Système d'imagerie par résonance magnétique supraconducteur pour une extrémité, comprenant un système magnétique (1), un système à radiofréquence et une console (10), le système magnétique (1) comprenant un boîtier principal (2) en forme de boucle, le système à radiofréquence comprenant des bobines à radiofréquence en forme de boucle, les bobines à radiofréquence comprenant une bobine d'extrémité (4) et au moins deux types de bobine de partie d'extrémité, la bobine d'extrémité (4) étant une bobine d'émissionréception, et chacune des bobines de partie d'extrémité étant une bobine uniquement de réception réalisée de manière à uniquement recevoir des signaux ; la bobine d'extrémité (4) étant agencée sur une surface intérieure en forme de boucle du boîtier principal (2), chacune des bobines de partie d'extrémité étant réalisée de manière à être reliée de manière commutable à une surface intérieure en forme de boucle de la bobine d'extrémité (4), et les bobines à radiofréquence étant reliées de manière communicative et amovible à la console (10),
les bobines à radiofréquence comprenant en outre une bobine de genou (5) pour l'imagerie d'un genou, la bobine de genou (5) étant une bobine d'émissionréception, la bobine d'extrémité (4) et la bobine de genou (5) étant réalisées de manière à être reliées de manière commutable à la surface intérieure en forme de boucle du boîtier principal (2), et un diamètre intérieur de la bobine de genou (5) étant inférieur à un diamètre intérieur de la bobine d'extrémité (4), et
les bobines de partie d'extrémité comprenant une bobine de main (6) pour l'imagerie d'une main, la bobine de main (6) comprenant un corps principal (61) de bobine de main en forme de boucle et une plaque de support (62) de bobine de main qui est agencée d'un côté axial du corps principal (61) de la bobine de main, une surface intérieure en forme de boucle du corps principal (61) de la bobine de main étant formée par une surface en forme de C et une surface plane de corps principal reliée à une ouverture de la surface en forme de C, et la plaque de support (62) de la bobine de la main étant une surface plane et étant agencée de manière coplanaire par rapport à la surface plane du corps principal.

2. Système d'imagerie par résonance magnétique supraconducteur pour une extrémité selon la revendication 1, un diamètre de la surface intérieure en forme de boucle de la bobine d'extrémité (4) étant compris dans une plage de 175 mm à 185 mm, et un diamètre d'une surface de diamètre intérieure de la bobine de genou (5) étant compris dans une plage de 155 mm à 165 mm.

3. Système d'imagerie par résonance magnétique supraconducteur pour une extrémité selon la revendication 1, les bobines de partie d'extrémité comprenant en outre au moins une bobine de coude (7) pour l'imagerie d'un coude et une bobine de cheville (8) pour l'imagerie d'une cheville.

4. Système d'imagerie par résonance magnétique supraconducteur pour une extrémité selon la revendication 3, la bobine de coude (7) comprenant un corps principal (71) de bobine de coude en forme de boucle et deux plaques de support de bobine de coude agencées de deux côtés axiaux du corps principal (71) de la bobine de coude.

5. Système d'imagerie par résonance magnétique supraconducteur pour une extrémité selon la revendication 3, la bobine de cheville (8) comprenant un corps principal (81) de bobine de cheville en forme de boucle et une plaque de support (82) de bobine de cheville en forme de boucle, un orifice du corps principal (81) de la bobine de cheville étant en butée contre un orifice de la plaque de support (82) de la bobine de cheville, et un angle inclus étant formé entre une ligne médiane du corps principal (81) de la bobine de cheville et une ligne médiane de la plaque de support (82) de la bobine de cheville.

6. Système d'imagerie par résonance magnétique supraconducteur pour une extrémité selon l'une des revendications 1 à 5, comprenant en outre un fauteuil de scannage (9) qui est fabriqué en un matériau non-magnétique et est agencé à l'extérieur du boîtier principal (2), le fauteuil de scannage (9) comprenant une plaque d'assise (93), un appui-pieds (91) et un appui-dos (92) agencés de deux côtés de la plaque d'assise (93), et un angle de l'appui-pieds (91) et un angle de l'appui-dos (92) étant réglables par rapport à la plaque d'assise (93).

7. Système d'imagerie par résonance magnétique supraconducteur pour une extrémité selon la revendication 6, le système magnétique (1) comprenant en outre un aimant supraconducteur agencé dans le boîtier principal (2), une intensité de champ magnétique de l'aimant supraconducteur étant égale à 3,0 T, et une séquence d'imagerie dédiée aux extrémités de 3,0 T étant intégrée dans un contrôleur de la console (10).

8. Système d'imagerie par résonance magnétique supraconducteur pour une extrémité selon la revendication 6, le système magnétique (1) comprenant en outre un système à gradient (3), le système à gradient (3) comprenant une bobine à gradient, la bobine à gradient présentant une structure annulaire d'une longueur inférieure ou égale à 640 mm, un diamètre extérieur inférieur ou égal à 294 mm, et un diamètre intérieur supérieur ou égal à 210 mm.
